# EUROPÄISCHE PATENTSCHRIFT

(11) **EP 1 017 670 B1**
(45) Veröffentlichungstag und Bekanntmachung des Hinweises auf die Patenterteilung: **27.11.2002**
(21) Anmeldenummer: 98947518.1
(22) Anmeldetag: 05.09.1998
(51) Int. Cl.: C07C 259/18, C07D 333/24, C07D 231/12, C07C 255/53, C07C 255/54, A01N 37/52, A01N 43/10, A01N 43/56

(54) **BENZAMIDOXIM-DERIVATE, ZWISCHENPRODUKTE UND VERFAHREN ZU DEREN HERSTELLUNG UND DEREN VERWENDUNG ALS FUNGIZIDE**
BENZAMIDOXIM DERIVATIVES, INTERMEDIATE PRODUCTS AND METHODS FOR PREPARING AND USING THEM AS FUNGICIDES
DERIVES DE BENZAMIDOXIME, PRODUITS INTERMEDIAIRES ET PROCEDES POUR LES PREPARER ET LES UTILISER COMME FONGICIDES

(30) Priorität: 18.09.1997 DE 19741099; 03.12.1997 DE 19753519; 23.01.1998 DE 19802459
(43) Veröffentlichungstag der Anmeldung: 12.07.2000
(73) Patentinhaber: BASF AKTIENGESELLSCHAFT, 67056 Ludwigshafen (DE)
(72) Erfinder: EICKEN, Karl, D-67157 Wachenheim (DE); RHEINHEIMER, Joachim, D-67063 Ludwigshafen (DE); WETTERICH, Frank, D-67112 Mutterstadt (DE); AMMERMANN, Eberhard, D-64646 Heppenheim (DE); LORENZ, Gisela, D-67434 Neustadt (DE); STRATHMANN, Siegfried, D-67117 Limburgerhof (DE)
(86) Internationale Anmeldenummer: EP9805617
(87) Internationale Veröffentlichungsnummer: WO99014187

(56) Entgegenhaltungen:
- EP-A- 0 353 631
- EP-A- 0 402 751
- EP-A- 0 490 224
- EP-A- 0 624 567
- PATENT ABSTRACTS OF JAPAN vol. 98, no. 1, 30. Januar 1998 & JP 09 235262 A (NIPPON SODA CO LTD), 9. September 1997
- PATENT ABSTRACTS OF JAPAN vol. 13, no. 224 (C-599), 24. Mai 1989 & JP 01 034954 A (HOKKO CHEM IND CO LTD), 6. Februar 1989
- PATENT ABSTRACTS OF JAPAN vol. 14, no. 134 (C-0701), 14. März 1990 & JP 02 006453 A (NIPPON SODA CO LTD), 10. Januar 1990 in der Anmeldung erwähnt
- PATENT ABSTRACTS OF JAPAN vol. 13, no. 532 (C-659), 28. November 1989 & JP 01 215994 A (HOKKO CHEM IND CO LTD), 29. August 1989

## Beschreibung

Die vorliegende Erfindung betrifft neue Benzamidoxim-Derivate, Verfahren und Zwischenprodukte zu deren Herstellung und deren Verwendung als Fungizide.

Aus der JP-A 09/235262 und der JP-A 02/006453 sind Benzamidoxim-Derivate mit fungizider Wirksamkeit beschrieben, die jedoch insbesondere bei niedrigen Aufwandmengen nicht in vollem Umfang zufrieden stellen können. EP-A-0624567 offenbart neue Derivate des 3-Fluorphenols.

Aufgabe der vorliegenden Erfindung war es daher, neue Benzamidoxim-Derivate mit verbesserter Wirkung, insbesondere auch bei niedrigen Aufwandmengen, zur Verfügung zu stellen.

Überraschenderweise wurde nun gefunden, daß Benzamidoxim-Derivate der Formel I 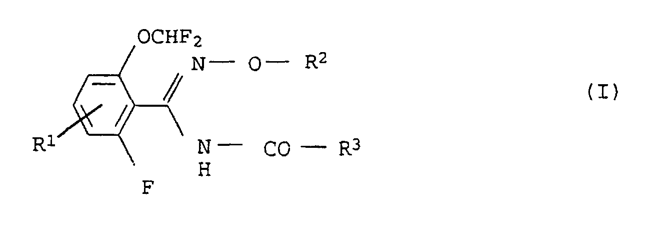 wobei die Substituenten die folgenden Bedeutungen haben:
- R¹: Wasserstoff oder Fluor
- R²: C₁-C₄-Alkyl, welches durch Cyano substituiert sein kann, C₁-C₄-Halogenalkyl, C₁-C₄-Alkoxy-C₁-C₄-alkyl, C₃-C₆-Alkenyl, C₃-C₆-Halogenalkenyl, C₃-C₆-Alkinyl, C₃-C₈-Cycloalkyl-C₁-C₄-Alkyl
- R³: Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder Thienyl-C₁-C₄-Alkyl, welches am Thienylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder Pyrazol-C₁-C₄-Alkyl, welches am Pyrazolring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann.

Bei der Definition der Substituenten R¹ bis R³ stehen die angegebenen Begriffe als Sammelbegriff für eine Gruppe von Verbindungen.

Halogen steht jeweils für Fluor, Brom, Chlor oder Iod, insbesondere für Fluor oder Chlor.

Ferner stehen beispielsweise:
- C₁-C₄-Alkyl für: Methyl, Ethyl, n-Propyl, 1-Methylethyl, n-Butyl, 1-Methylpropyl, 2-Methylpropyl oder 1,1-Dimethylethyl, insbesondere für Ethyl;
- C₁-C₄-Halogenalkyl für: einen C₁-C₄-Alkylrest wie vorstehend genannt, der partiell oder vollständig durch Fluor, Chlor, Brom und/oder Iod substituiert ist, z.B. Trichlormethyl, Trifluormethyl, 2-Fluorethyl, 2-Chlorethyl, 2-Bromethyl, 2,2-Difluorethyl, 2,2,2-Trifluorethyl, 2,2,2-Trichlorethyl, 2-Fluorpropyl, 3-Fluorpropyl, 2-Chlorpropyl oder 3-Chlorpropyl, insbesondere für 2-Fluorethyl oder 2-Chlorethyl;
- Cyano-C₁-C₄-alkyl für: z.B. Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl oder 2-Cyanoprop-2-yl, insbesondere für Cyanomethyl oder 2-Cyanoethyl;
- C₁-C₄-Alkoxy für: Methoxy, Ethoxy, n-Propoxy, 1-Methylethoxy, n-Butoxy, 1-Methylpropoxy, 2-Methylpropoxy oder 1,1-Dimethylethoxy, insbesondere für Methoxy oder Ethoxy;
- C₁-C₄-Alkoxy-C₁-C₄-alkyl für: durch C₁-C₄-Alkoxy wie vorstehend genannt substituiertes C₁-C₄-Alkyl, also z.B. für Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, 2-(Methoxy)ethyl oder 2-(Ethoxy)ethyl, insbesondere für Methoxymethyl oder 2-Methoxyethyl;
- C₃-C₆-Alkenyl für: z.B. Prop-2-en-1-yl, n-Buten-4-yl, 1-Methyl-prop-2-en-1-yl, 2-Methyl-prop-2-en-1-yl oder 2-Buten-1-yl, insbesondere für Prop-2-en-1-yl;
- C₃-C₆-Halogenalkenyl für: C₃-C₆-Alkenyl wie vorstehend genannt, das partiell oder vollständig durch Fluor, Chlor und/oder Brom substituiert ist, z.B. 2-Chlorallyl, 3-Chlorallyl, 2,3-Dichlorallyl oder 3,3-Dichlorallyl, insbesondere für 2-Chlorallyl;
- C₃-C₆-Alkinyl für: z.B. Prop-1-in-1-yl, Prop-2-in-1-yl, n-But-1-in-1-yl, n-But-1-in-3-yl, n-But-1-in-4-yl oder n-But-2-in-1-yl, insbesondere für Prop-2-in-1-yl;
- C₃-C₈-Cycloalkyl-C₁-C₄-alkyl für: z.B. Cyclopropylmethyl, Cyclobutylmethyl, Cyclopentylmethyl, Cyclohexylmethyl, (Cyclopropyl)ethyl, 1-(Cyclobutyl)ethyl, 1-(Cyclopentyl)-ethyl, 1-(Cyclohexyl)ethyl, 1-(Cycloheptyl)ethyl, 1-(Cyclooctyl)ethyl, 2-(Cyclopropyl)ethyl oder 2-(Cyclobutyl)ethyl, insbesondere für Cyclopentylmethyl;
- Phenyl-C₁-C₆-alkyl für: z.B. Benzyl, 1-Phenylethyl, 2-Phenylethyl, 1-Phenylprop-1-yl, 2-Phenylprop-1-yl, 3-Phenylprop-1-yl, insbesondere für Benzyl oder 2-Phenylethyl;
- Thienyl-C₁-C₄-alkyl für: z.B. 2-Thienylmethyl, 3-Thienylmethyl oder 2-Thienylethyl;
- Pyrazol-C₁-C₄-alkyl für: z.B. 1-Pyrazolylmethyl, 2-Pyrazolylmethyl, 3-Pyrazolylmethyl oder 2-Pyrazolylylethyl;

Verbindungen in denen der Substituent R² für Cyclopropylmethyl und der Substituent R³ für Benzyl, welches am Phenylring einen bis drei Substituenten ausgewählt aus der vorstehend genannten Gruppe, insbesondere einen bis drei Substituenten ausgewählt aus Fluor, Chlor, Methyl, Methoxy oder Trifluormethyl, steht, haben sich als in der Regel besonders wirksam erwiesen.

Verbindungen der Formel I, in denen R¹ bis R³ die in der nachstehenden Tabelle 1 aufgeführten Bedeutungen haben, sind insbesondere bevorzugt.

**Tabelle 1**

| **Nr.** | **R**^{**1**} | **R**^{**2**} | **R**^{**3**} | **Fp.°C** |
|---|---|---|---|---|
| I.1 | H | C₂H₅ | C₆H₅-CH₂ | Öl |
| I.2 | H | C₂H₅ | 4-CH₃O-C₆H₄-CH₂ | Öl |
| I.3 | H | CH₂-CH=CH₂ | C₆H₅-CH₂ | Öl |
| I.4 | H | CH₂-C≡CH | C₆H₅-CH₂ | Öl |
| I.5 | H | CH₂-C≡CH | 4-CH₃O-C₆H₄-CH₂ | Öl |
| I.6 | H | cPr | C₆H₅-CH₂ | |
| I.7 | H | cPr | 4-F-C₆H₄-CH₂ | 75-77 |
| I.8 | H | cPr | 4-Cl-C₆H₄-CH₂ | 81-83 |
| I.9 | H | cPr | 4-CH₃O-C₆H₄-CH₂ | 57-59 |
| I.10 | H | cPr | 4-CF₃-C₆H₄-CH₂ | |
| I.11 | H | cPr | 2-Thienylmethyl | Öl |
| I.12 | H | cPr | 3-Thienylmethyl | Öl |
| I.13 | H | cPr | Pyrazolyl-1-methyl | |
| I.14 | H | cPr | 4-CH₃-C₆H₄-CH₂ | |
| I.15 | 5-F | CH₂-CH=CH₂ | C₆H₅-CH₂ | |
| I.16 | 5-F | CH₂-CH=CH₂ | 4-CH₃-C₆H₄-CH₂ | |
| I.17 | 5-F | CH₂-C≡CH | C₆H₅-CH₂ | |
| I.18 | 5-F | CH₂-C≡CH | 4-CH₃O-C₆H₄-CH₂ | |
| I.19 | 5-F | cPr | C₆H₅-CH₂ | 62-65 |
| I.20 | 5-F | cPr | 4-F-C₆H₄-CH₂ | 64-67 |
| I.21 | 5-F | cPr | 4-Cl-C₆H₄-CH₂ | 72-75 |
| I.22 | 5-F | cPr | 4-CH₃-C₆H₄-CH₂ | 74-76 |
| I.23 | 5-F | cPr | 4-CH₃O-C₆H₄-CH₂ | 79-81 |
| I.24 | 5-F | cPr | 4-CF₃-C₆H₄-CH₂ | |
| I.25 | 4-F | cPr | C₆H₅-CH₂ | |
| I.26 | 4-F | cPr | 4-CH₃O-C₆H₄-CH₂ | |
| I.27 | H | cPr | 4-CH₃-C₆H₄-CH₂ | 69-71 |
| I.28 | H | CH₂-C≡CH | 4-F-C₆H₄-CH₂ | 74-76 |
| I.29 | H | C₂H₅ | 4-F-C₆H₄-CH₂ | Öl |
| I.30 | H | CH₂-CH=CH₂ | 4-F-C₆H₄-CH₂ | Öl |
| I.31 | H | CH₂-CH=CH₂ | 4-CH₃O-C₆H₄-CH₂ | Öl |
| I.32 | H | CH₂-CH(CH₃)₂ | C₆H₅-CH₂ | 65-67 |
| I.33 | H | CH₂-C(CH₃)=CH₂ | C₆H₅-CH₂ | Öl |

cPr steht in der vorstehenden Tabelle für Cyclopropylmethyl

Die erfindungsgemäßen Benzamidoxim-Derivate der Formel I erhält man nach dem erfindungsgemäßen Verfahren durch Etherspaltung von fluorierten Benzonitrilen der Formel II, Umsetzung der erhaltenen Benzonitrile III mit Halogenalkanen CHF₂Hal wie CHF₂Cl in basischem Medium (vorzugsweise in Gegenwart eines Alkalimetallhydroxids) zu Benzonitrilen IV und anschließende Umsetzung von IV mit Hydroxylamin oder dessen Salzen in wäßriger Lösung, vorzugsweise in Wasser oder Wasser/Alkanol-Gemischen, gegebenenfalls in Anwesenheit einer Base zu den Benzamidoximen der Formel V, die dann anschließend in an sich bekannter Weise zu den Vorprodukten VI alkyliert werden.

Die Benzamidoxime VI können dann in an sich bekannter Weise mit den entsprechenden Säurehalogeniden , vorzugsweise den entsprechenden Säurechloriden, durch Erwärmen in inerten Lösungsmitteln (vorzugsweise auf Temperaturen im Bereich von 20 bis 100 °C) acyliert werden.

Als inerte Lösungsmittel eignen sich insbesondere Kohlenwasserstoffe oder Ether, besonders bevorzugt aromatische Kohlenwasserstoffe wie Toluol oder Xylol, um nur zwei Beispiele zu nennen.

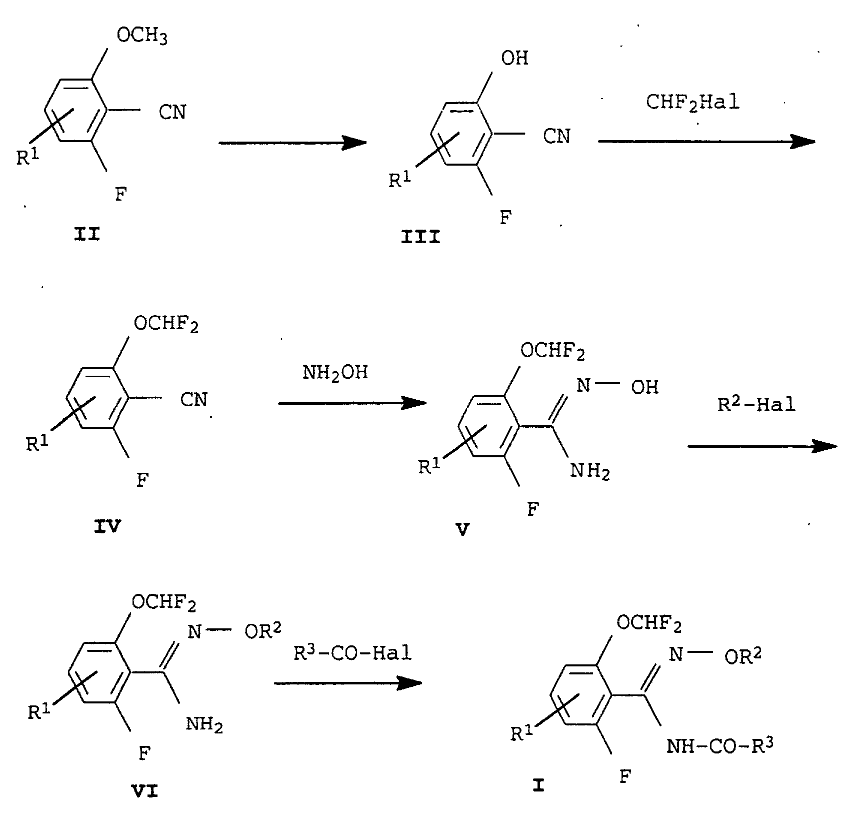

Die in vorstehendem Reaktionsschema aufgeführten Zwischenprodukte der Formel III, in denen R¹ für Fluor steht und die Zwischenprodukte der Formeln IV, V und VI sind neu und stellen einen weiteren Gegenstand der Erfindung dar.

Die Herstellung dieser neuen Zwischenprodukte mit einer Difluorsubstitution kann, ausgehend von 2,3-Difluor-6-methoxybenzaldehyd (welches z.B. nach dem Verfahren des Beispiels 27 der WO 97/03071 hergestellt werden kann) nach den in Beispiel 2 beschriebenen Varianten bis zur Stufe der Verbindungen IV geführt werden. Die weiteren Schritte zur Herstellung der entsprechenden Verbindungen V und VI sind dem Fachmann an sich bekannt.

Bevorzugte Verbindungen der Formeln IV, V und VI sind solche, in denen R¹ und R² (Verbindungen VI) die vorstehend für Verbindungen der Formel I genannten Bedeutungen haben.

Bevorzugte Verbindungen der Formel IV sind die in der nachstehenden Tabelle 2 aufgeführten Verbindungen.

| Nr. | R¹ | Fp.[°C], NMR (CDCl₃) ppm |
|---|---|---|
| II.1 | H | 6,7t(1H);7,05-7,2m(2H);7,55-7,7m(1H) |
| II.2 | 5-F | 6,65t(1H); 7,05-7,2m(1H); 7,4-7,5m(1H) |
| II.3 | 4-F | |

Bevorzugte Verbindungen der Formel V sind in der Tabelle 3 aufgeführt.

| Nr. | R¹ | Fp.[°C], NMR (CDCl₃) ppm |
|---|---|---|
| III.1 | H | 5,9s(2H);7,0-7,2m(2H);7,15t(1H);7,4-7,55m (1H) |
| III.2 | 5-F | 4,9s(2H);6,5t(1H); 6,95-7,05m(1H); 7,15-7,3m(1H); 8,0s(1H) |
| III.3 | 4-F | |

In der nachstehenden Tabelle 4 sind einige bevorzugte Verbindungen der Formel VI aufgeführt.

| Nr. | R¹ | R² | Fp.[°C], NMR (CDCl₃) ppm |
|---|---|---|---|
| IV.1 | H | C₂H₅ | |
| IV.2 | H | CH₂-CH=CH₂ | |
| IV.3 | H | CH₂-C≡CH | |
| IV.4 | H | c-Pr | 0,3m(2H);0,55m(2H);1,2m(1H); 3,9d(2H);4,85s,br(2H);6,6t(1H);6,85-7,1m (2H);7,35-7,45m(1H) |
| IV.5 | 5-F | C₂H₅ | |
| IV.6 | 5-F | CH₂-CH=CH₂ | |
| IV.7 | 5-F | CH₂-C≡CH | |
| IV.8 | 5-F | c-Pr | 0,3m(2H); 0,55m(2H); 1,2m(1H); 3,85d(2H); 4,9s br(2H); 6,55t(1H); 7,0-7,1m(1H); 7,15-7,2m(1H) |

Die Verbindungen I zeichnen sich durch eine hervorragende Wirkung gegen ein breites Spektrum von pflanzenpathogenen Pilzen, insbesondere aus der Klasse der Ascomyceten, Deuteromyceten, Phycomyceten und Basidiomyceten, aus. Sie sind z.T. systemisch wirksam und können daher auch als Blatt- und Bodenfungizide eingesetzt werden.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-iso-butylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Deuteromyceten, Ascomyceten, Phycomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycärbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethylfuran-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-l-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl) oxiran-2-yl-methyl]-1H-1,2,4-triazol sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl-l,3-oxazolidin-2,4-dion, 3-(3,5-Dichlorphenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoximino-[α-(o-tolyloxy)-o-tolyl]acetat, Methyl-E-2-{2-[6-(2-cyanophenoxy)pyridimin-4-yloxy]phenyl}-3-methoxyacrylat, Methyl-E-methoximino-[α-(2,5-dimethyloxy)-o-tolyl]acetamid.

Anilino-Pyrimidine wie N-(4,6-dimethylpyrimidin-2-yl)anilin, N-[4-methyl-6-(1-propinyl)pyrimidin-2-yl]anilin, N-(4-methyl-6-cyclopropyl-pyrimidin-2-yl)anilin.

Phenylpyrrole wie 4-(2,2-difluor-1,3-benzodioxol-4-yl)-pyrrol-3-carbonitril.

Zimtsäureamide wie 3-(4-chlorphenyl)-3-(3,4-dimethoxyphenyl)-acrylsäuremorpholid.

### Beispiel 1

### a) 6-Fluor-2-hydroxybenzonitril

7,8 g 2-Methoxy-6-fluorbenzonitril und 18,0 g Pyridinhydrochlorid wurden unter trockenem Stickstoff 5 h auf 195 °C erhitzt. Nach dem Abkühlen wurde der Ansatz zwischen 50 ml Wasser und 50 ml tert.-Butylmethylether verteilt und anschließend die organische Phase mit 40 ml 2n NaOH extrahiert. Der Alkaliextrakt wurde auf pH 5 eingestellt und anschließend zweimal mit je 40 ml tert.-Butylmethylether extrahiert. Nach dem Verdampfen des Lösungsmittels wurden 4,7 g des gewünschten Produkts als Öl erhalten (HPLC: 93%).
NMR(DMSO) ppm: 6,8-6,95 m(2H); 7,5-7,6 m(1H); 11,8 s,br(1H).

### b) 2-Difluormethoxy-6-fluorbenzonitril

In eine Mischung von 10,0 g 2-Hydroxy-6-fluorbenzonitril, 50 ml 1,2-Dimethoxyethan und 25 ml NaOH (33 %) wurden unter Rühren bei 75 °C 6,3, g Chlordifluormethan eingegast (dabei wurde der Rückflußkühler mit Trockeneis gekühlt) und eine Stunde bei 70-75 °C nachgerührt. Nach dem Abkühlen wurde der Ansatz mit 300 ml Wasser verdünnt und dreimal mit je 150 ml tert.-Butylmethylether extrahiert. Nach dem Verdampfen des Lösungsmittels wurden 6,5 g des gewünschten Produkts als Öl erhalten.
NMR(CDCl₃) ppm: 6,7 t(1H); 7,05-7,20 m(2H); 755-7,70 m(1H).

### c) 2-Difluormethoxy-6-fluorbenzamidoxim

Eine Mischung von 6,4 g 2-Difluormethoxy-6-fluorbenzonitril und 3,1 g Hydroxylamin-Hydrochlorid, 2,6 g Natriumcarbonat, 7 ml Wasser und 35 ml Ethanol wurde 20 h bei 75 °C gerührt.

Nach dem Verdampfen des Lösungsmittels wurde der Rückstand zwischen 40 ml 2n HCl und 20 ml Ethylacetat verteilt. Nach dem Abtrennen der HCl-Phase, Neutralisation auf pH 7 und dreimaligem Extrahieren mit je 40 ml tert.-Butylmethylether wurde das Lösungsmittel verdampft. Man erhielt 6,0 g des gewünschten Produkts.
NMR(DMSO) ppm: 5,9 s(2H); 7,0-7,2 m(2H); 7,15 t(1H), 7,4-7,44 m(1H); 9,5 s(1H).

### d) 2-Difluormethoxy-6-fluorbenzamid-[O-cyclopropylmethyl]-oxim

Zu einer Lösung von 3,0 g 2-Difluormethoxy-6-fluorbenzamidoxim in 30 ml Dimethylformamid (DMF) wurden bei 0 bis 5 °C 0,4 g 80%iges Natriumhydrid gegeben und bei dieser Temperatur 3 h gerührt. Anschließend wurden bei der gleichen Temperatur 1,8 g Bromcyclopropylmethan zugegeben und anschließend 2 h bei 5 °C und über Nacht bei Raumtemperatur nachgerührt. Der Ansatz wurde in 300 ml Wasser eingerührt und dreimal mit je 70 ml Cyclohexan extrahiert. NAch Verdampfen des Cyclohexans wurden 1,9 g des gewünschten Produkts erhalten.
NMR(CDCl₃) ppm: 0,3 m(2H); 0,55 m(2H); 1,2 m(1H); 3,9 d(2H); 4,85 s,br(2H); 6,6 t(1H); 6,85-7,1 m(2H); 7,35-7,45 m(1H).

### e) N-Phenylacetyl-2-difluormethoxy-6-fluorbenzamid-[O-cyclopropylmethyl]-oxim (Verbindung I.6 aus Tabelle 1).

1,9 g des nach Stufe d) erhaltenen 2-Difluormethoxy-6-fluorbenzamid-[O-cyclopropylmethyl]-oxims und 1,5 g Phenylessigsäurechlorid wurden mit 40 ml Toluol 20 h am Rückfluß erhitzt. Nach dem Abkühlen wurden 40 ml Wasser zugesetzt und auf pH 11 eingestellt.. Aus der Toluolphase wurden nach Verdampfen des Lösungsmittels und anschließender Säulecnchromatographie an Kieselgel mi.t einem 99:1 Gemisch aus Cyclohexan und Etylacetat als Laufmittel 1, 6 g des gewünschten Produkts mit einem Fp. von 58-60 °C isoliert.
NMR(CDCl₃) ppm: 0,2 m(2H); 0,50 m(2H); 1,0 m(1H); 3,9 d(2H); 6,4 t(1H); 6,85-7,0 m(2H); 7,2-7,5 m(6H); 8,5 s(1H).

Auf analoge Weise wurde N-Phenylacetyl-2-difluormethoxy-6-fluorbenzamid-[O-allylmethyl]-oxim (Verbindung I.3. aus Tabelle 1) als Öl erhalten.

### Beispiel 2 - Herstellung von 2-Hydroxy-5,6-difluorbenzonitril

### a) Herstellung von 2-Methoxy-5,6-difluorbenzaldehydoxim

Zu einer Mischung von 16,0 g Hydroxylaminhydrochlorid, 18,9 g Natriumacetat und 110 ml 90%igem wäßrigem Methanol tropfte man unter Rühren bei 20-25°C eine Lösung von 29,4 g 2-Methoxy-5,6-difluorbenzaldehyd (Gemäß Bsp. 27 der WO 97/03071). Nach 16 Stunden Rühren wurden nach Verdampfen des Methanols, Anteigen mit 250 ml Wasser, Waschen und Trocknen 28,3 g des gewünschten Produkts mit einem Fp. von 199-201°C erhalten.

### b) Herstellung von 2-Methoxy-5,6-difluorbenzonitril

Zu einer Suspension von. 18,7 g des nach a) erhaltenen Produkts in 100 ml Toluol wurden 20 Tropfen Dimethylformamid gegeben und 16,6 g Thionylchlorid gegeben, wobei darauf geachtet wurde, daß die Temperatur nicht höher als 30 °C stieg. Nach 4 Stunden Rühren bei 30 °C, Verdampfen von Toluol und Thionylchlorid im Vakuum wurden 16,5 g des gewünschten Produkts als Öl isoliert. (NMR(CDCl₃) ppm: 3,9s(3H); 6,65-6,75m(1H); 7,3-7,45m(1H).

### c) Herstellung von 2-Hydroxy-5,6-difluorbenzonitril

Zu einer Lösung von 23,0 g des nach b) erhaltenen Produkts in 70 ml Toluol wurden bei 50 °C unter Rühren portionsweise 21,7 g AlCl₃ gegeben. Nach Ende der Zugabe wurde 2 Stunden zum Rückfluß erhitzt. Nach dem Abkühlen wurde das Reaktionsgemisch auf 350 ml Wasser gegossen und mit 2n HCl auf einen pH-Wert von 1 gebracht. Das so erhaltene Rohprodukt wurde mit tert.Butylmethylether (2 x 100 ml) extrahiert und durch Lösen in 2n NaOH (2 x 80 ml) und Ansäuern der alkalischen Phase mit 2n Hcl auf pH 5 gereinigt. Nach Extraktion mit tert.Butylmethylether (2x 80 ml), Trocknen und Verdampfen des Lösungsmittels wurden 19,9 g des.gewünschten.. Produkts als Öl isoliert. (NMR (CDCl₃) ppm: 6,45s,br(1H); 6,7-6,8m(1H); 7,25-7,4m(1H).

### Beispiel 3

### Wirksamkeit gegen Weizenmehltau

Blätter von in Töpfen gewachsenen Weizenkeimlingen der Sorte "Frühgold" wurden mit wäßriger Wirkstoffaufbereitung, die aus einer Stammlösung bestehend aus 10 % Wirkstoff, 63% Cyclohexanon und 27 % Emulgiermittel angesetzt wurde, bis zur Tropfnässe besprüht und 24 h nach dem Antrocknen des Spritzbelages mit Sporen des Weizenmehltaus (Erysiphe graminis f.sp.tritici) bestäubt. Die Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung visuell in % der gesamten Blattfläche ermittelt.

Die mit den Wirkstoffen I.3, I.6, I.7, I.8, I.9. I.11, I.12 und I.27. der Tabelle 1 mit 63 ppm-haltiger wäßriger Wirkstoffaufbereitung behandelten Pflanzen zeigten keinen Befall, während die unbehandelten Pflanzen zu 80 % befallen waren.

### Beispiel 4 : Wirksamkeit gegen Weizenmehltau

Blätter von in Töpfen gewachsenen Weizenkeimlingen der Sorte "Frühgold" wurden mit wäßriger Wirkstoffaufbereitung, die aus einer Stammlösung bestehend aus 10 % Wirkstoff, 63% Cyclohexanon und 27 % Emulgiermittel angesetzt wurde, bis zur Tropfnässe besprüht und 24 h nach dem Antrocknen des Spritzbelages mit Sporen des Weizenmehltaus (Erysiphe graminis f.sp.tritici) bestäubt. Die Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung visuell in % der gesamten Blattfläche ermittelt.

Die mit den Wirkstoffen II.1 und II.3 der Tabelle 2 behandelten Pflanzen zeigten keinen Befall, während die unbehandelten Pflanzen zu 80 % befallen waren.

## Patentansprüche

1. Benzamidoxim-Derivate der Formel I 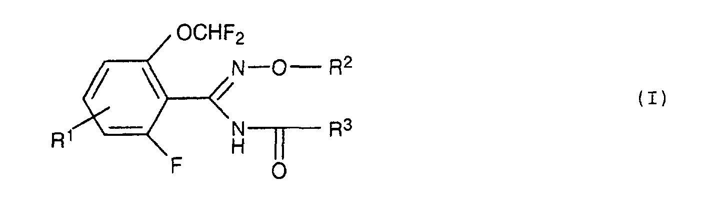 wobei die Substituenten die folgenden Bedeutungen haben:
R¹ Wasserstoff oder Fluor
R² C₁-C₄-Alkyl, welches durch Cyano substituiert sein kann, C₁-C₄-Halogenalkyl, C₁-C₄-Alkoxy-C₁-C₄-alkyl, C₃-C₆-Alkenyl, C₃-C₆-Halogenalkenyl, C₃-C₆-Alkinyl, C₃-C₈-Cycloalkyl-C₁-C₄-Alkyl
R³ Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches am Thienylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazol-C₁-C₄-Alkyl, welches am Pyrazolring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann.

2. Benzamidoxim-Derivate der Formel I nach Anspruch 1, in der R³ für Benzyl steht, welches am Phenylring einen bis drei Substituenten ausgewählt aus der Gruppe bestehend aus Halogen, C₁-C₄-Alkyl, oder C₁-C₄-Alkoxy tragen kann.

3. Benzamidoxim-Derivate der Formel I nach Anspruch 1, wobei R¹ Fluor ist und an 5-Position des Phenylringes steht.

4. Benzonitrile der Formel IV 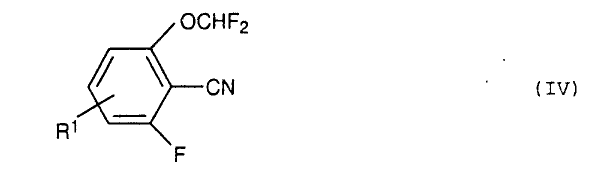 wobei R¹ die in Anspruch 1 genannte Bedeutung hat.

5. Benzonitrile der Formel IV nach Anspruch 4, wobei R¹ Fluor ist und an 5-Position des Phenylringes steht.

6. Benzamidoxime der Formel V 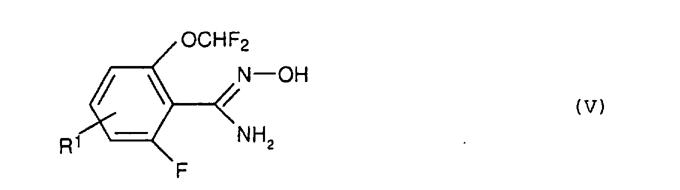 wobei R¹ die in Anspruch 1 genannte Bedeutung hat.

7. Benzamidoxime der Formel V nach Anspruch 7, wobei R¹ Fluor ist und an 5-Position des Phenylringes steht.

8. Benzamidoxime der Formel VI 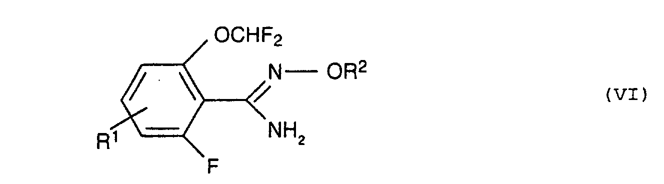 wobei R¹ und R² die in Anspruch 1 genannte Bedeutung haben.

9. Benzamidoxime der Formel VI nach Anspruch 8, wobei R¹ Fluor ist und an 5-Position des Phenylringes steht.

10. Verwendung der Benzamidoxim-Derivate der Formel I gemäß einem der Ansprüche 1 - 3 zur Bekämpfung von Schadpilzen.

11. Fungizide Mittel, enthaltend eine fungizid wirksame Menge mindestens eines Benzamidoxim-Derivats der Formel I gemäß einem der Ansprüche 1 - 3.

12. Verfahren zur Bekämpfung von Schadpilzen, **dadurch gekennzeichnet, daß** man die Schadpilze, deren Lebensraum oder die. von ihnen freizuhaltenden Pflanzen, Flächen,. Materialien oder Räume mit einer fungizid wirksamen Menge einer Verbindung der allgemeinen Formel I oder einem ein Benzamidoxim-Derivat der Formel I enthaltenden fungiziden Mittel gemäß Anspruch 11 behandelt.

## Claims

1. A benzamidoxime derivative of the formula I 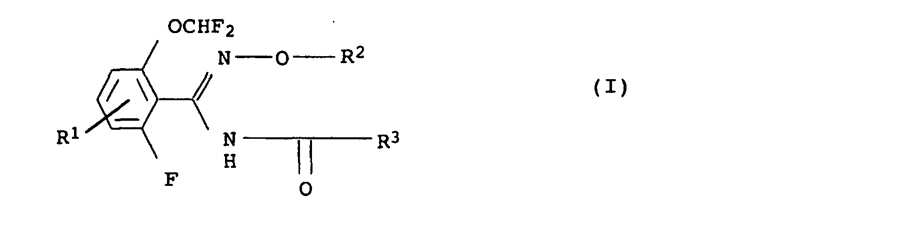 where:
R¹ is hydrogen or fluorine
R² is C₁-C₄-alkyl which may be substituted by cyano, C₁-C₄-haloalkyl, C₁-C₄-alkoxy-C₁-C₄-alkyl, C₃-C₆-alkenyl, C₃-C₆-haloalkenyl, C₃-C₆-alkynyl, C₃-C₈-cycloalkyl-C₁-C₄-alkyl
R³ is phenyl-C₁-C₆-alkyl which may carry one or more substitutents selected from the group consisting of halogen, C₁-C₄-alkyl, C₁-C₄-haloalkyl, C₁-C₄-alkoxy or C₁-C₄-haloalkoxy on the phenyl ring, or
is thienyl-C₁-C₄-alkyl which may carry one or more substituents selected from the group consisting of halogen, C₁-C₄-alkyl, C₁-C₄-haloalkyl, C₁-C₄-alkoxy or C₁-C₄-haloalkoxy on the thienyl ring, or
is pyrazolyl-C₁-C₄-alkyl which may carry one or more substituents selected from the group consisting of halogen, C₁-C₄-alkyl, C₁-C₄-haloalkyl, C₁-C₄-alkoxy or C₁-C₄-haloalkoxy on the pyrazole ring.

2. A benzamidoxime derivative of the formula I as claimed in claim 1, in which R³ is benzyl which may carry one to three substituents selected from the group consisting of halogen, C₁-C₄-alkyl, or C₁-C₄-alkoxy on the phenyl ring.

3. A benzamidoxime derivative of the formula I as claimed in claim 1, where R¹ is fluorine and is in position 5 on the phenyl ring.

4. A benzonitrile of the formula IV 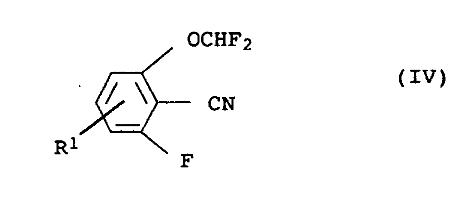 where R¹ is as defined in claim 1.

5. A benzonitrile of the the formula IV as claimed in claim 4, where R¹ is fluorine and is in position 5 on the phenyl ring.

6. A benzamidoxime of the formula V 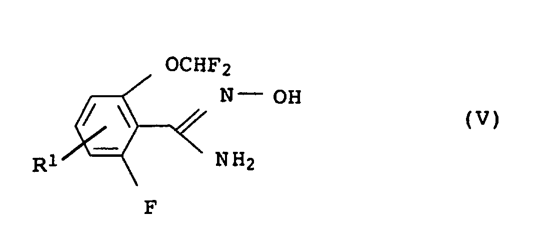 where R¹ is defined as in claim 1.

7. A benzamidoxime of the formula V as claimed in claim 7, where R¹ is fluorine and is in position 5 on the phenyl ring.

8. A benzamidoxime of the formula VI 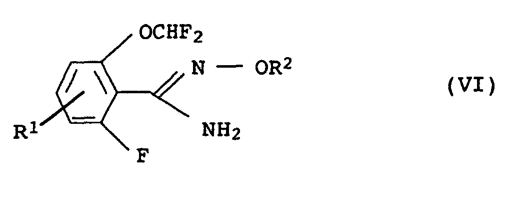 where R¹ and R² are as defined in claim 1.

9. A benzamidoxime of the formula VI as claimed in claim 8, where R¹ is fluorine and is in position 5 on the phenyl ring.

10. The use of the benzamidoxime derivative of the formula I as claimed in any of claims 1-3 for controlling harmful fungi.

11. A fungicidal composition, comprising a fungicidally effective amount of at least one benzamidoxime derivative of the formula I as claimed in any of claims 1-3.

12. A method for controlling harmful fungi, which comprises treating the harmful fungi, their habitat or the plants, areas, materials or spaces to be kept free from them with a fungicidally effective amount of a compound of the formula I or a fungicidal composition comprising a benzamidoxime derivative of the formula I as claimed in claim 11.

## Revendications

1. Dérivés de benzamidoxime de formule I 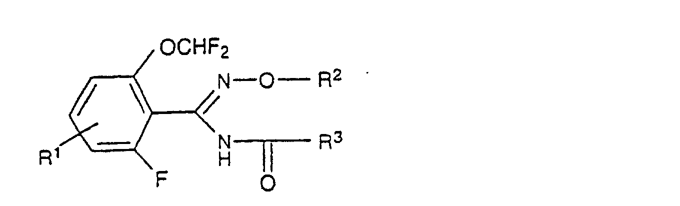 où les substituants ont les significations suivantes:
R¹ hydrogène ou fluor,
R² alkyle en C₁-C₄ -qui peut être substitué par un groupe cyano- halogénoalkyle en C₁-C₄, alcoxy(C₁-C₄)alkyle(C₁-C₄), alcényle en C₃-C₆, halogénoalcényle en C₃-C₆, alcynyle en C₃-C₆, cycloalkyl(C₃-C₈)alkyle(C₁-C₄),
R³ phényl-alkyle(C₁-C₆), qui peut porter sur le cycle phényle un ou plusieurs substituants choisis dans le groupe consistant en halogéno, alkyle en C₁-C₄, halogénoalkyle en C₁-C₄, alcoxy en C₁-C₄ ou halogénoalcoxy en C₁-C₄, ou
thiényl-alkyle(C₁-C₄), qui peut porter sur le cycle thiényle un ou plusieurs substituants choisis dans le groupe consistant en halogéno, alkyle en C₁-C₄, halogénoalkyle en C₁-C₄, alcoxy en C₁-C₄ ou halogénoalcoxy en C₁-C₄, ou
pyrazole-alkyle(C₁-C₄), qui peut porter sur le cycle pyrazole un ou plusieurs substituants choisis dans le groupe consistant en halogéno, alkyle en C₁-C₄, halogénoalkyle en C₁-C₄, alcoxy en C₁-C₄ ou halogénoalcoxy en C₁-C₄.

2. Dérivés de benzamidoxime de formule I selon la revendication 1, dans laquelle R³ désigne un benzyle, qui peut porter sur le cycle phényle un à trois substituants choisis dans le groupe consistant en halogéno, alkyle en C₁-C₄ ou alcoxy en C₁-C₄.

3. Dérivés de benzamidoxime de formule I selon la revendication 1, où R¹ est le fluor et se situe sur la position 5 du cycle phényle.

4. Benzonitriles de formule IV 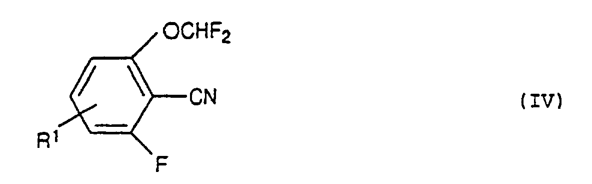 où R¹ a la signification indiquée dans la revendication 1.

5. Benzonitriles de formule IV selon la revendication 4, où R¹ est le fluor et se situe sur la position 5 du cycle phényle.

6. Benzamidoximes de formule V 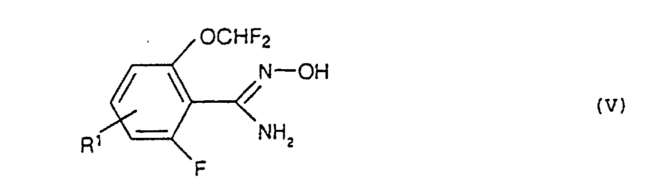 où R¹ a la signification indiquée dans la revendication 1.

7. Benzamidoximes de formule V selon la revendication 7, où R¹ est le fluor et se situe sur la position 5 du cycle phényle.

8. Benzamidoximes de formule VI 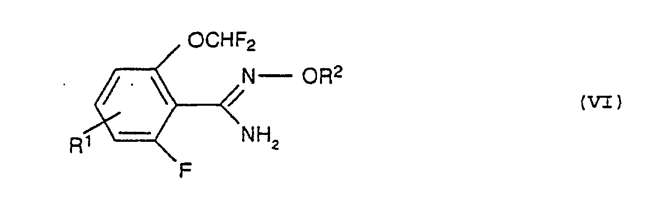 où R¹ et R² R¹ ont la signification indiquée dans la revendication 1.

9. Benzamidoximes de formule VI selon la revendication 8, où R¹ est le fluor et se situe sur la position 5 du cycle phényle.

10. Utilisation des dérivés de benzamidoxime de formule I selon l'une des revendications 1 - 3 pour la lutte contre les champignons nuisibles.

11. Produit fongicide, contenant une quantité à efficacité fongicide d'au moins un dérivé de benzamidoxime de formule I selon l'une des revendications 1 - 3.

12. Procédé pour la lutte contre les champignons nuisibles, **caractérisé par le fait qu'**on traite les champignons nuisibles, leur biotope ou les plantes, surfaces, matériaux ou espaces qui doivent en être exempts avec une quantité à efficacité fongicide d'un composé de formule générale I ou d'un produit fongicide contenant un dérivé de benzamidoxime de formule I selon la revendication 11.
